# DEMANDE DE BREVET EUROPEEN

(11) **EP 1 064 884 A1**
(43) Date de publication de la demande: **03.01.2001**
(21) Numéro de dépôt: 99401663.2
(22) Date de dépôt: 02.07.1999
(51) Int. Cl.: A61B 17/11, A61B 17/115, A61B 17/122

(54) **Système de liaison pour l'anastomose**

(71) Demandeur: LABORATOIRE PEROUSE IMPLANT, 60540 Bornel (FR)
(72) Inventeur: Zegdi, Rachid, 92140 Clamart (FR); Gerardin, Jean-Paul, 92250 La Garenne Colombes (FR); Perouse, Eric, 95290 L'Isle Adam (FR)
(74) Mandataire: Blot, Philippe Robert Emile

(57) **Abrégé**

L'invention concerne un organe de liaison (20) pour l'anastomose sur un conduit tubulaire (14) muni d'une ouverture latérale ou terminale, d'une prothèse vasculaire (12) comportant, à une extrémité de raccordement, un rebroussement (16) formant une nappe annulaire dont le bord libre est adapté pour s'appliquer au voisinage immédiat du pourtour de l'ouverture du conduit tubulaire. L'organe de liaison (20) comporte un ensemble d'attaches (22 ) adaptée chacune pour appliquer la nappe annulaire (16) sur le pourtour de l'ouverture. Il comporte en outre un support annulaire (26) le long duquel sont réparties les attaches (22).

Le dispositif de mise en place comporte un organe de liaison (20) et des moyens (24) de retenue des attaches en position ouverte. L'implant comporte une prothèse vasculaire (12) et un dispositif de mise en place.

## Description

La présente invention concerne un organe de liaison pour l'anastomose sur un conduit tubulaire muni d'une ouverture latérale ou terminale, d'une prothèse tubulaire comportant, à une extrémité de raccordement, un rebroussement formant une nappe annulaire dont le bord libre est adapté pour s'appliquer au voisinage immédiat du pourtour de l'ouverture du conduit tubulaire.

Elle concerne en outre un dispositif de mise en place d'attaches pour l'anastomose sur un conduit tubulaire muni d'une ouverture latérale ou terminale d'une prothèse tubulaire comportant, à une extrémité de raccordement, un rebroussement formant une nappe annulaire dont le bord libre est adapté pour s'appliquer au voisinage immédiat du pourtour de l'ouverture du conduit tubulaire.

Elle concerne enfin un implant comportant une prothèse tubulaire notamment une prothèse vasculaire, et un dispositif de mise en place d'un ensemble d'attaches.

Les prothèses vasculaires couramment utilisées à ce jour sont obtenues par tissage ou tricotage de fibres. Elles sont constituées par des conduits sensiblement cylindriques destinés à réaliser des pontages ou des substitutions de parties de conduits tubulaires malades.

L'anastomose d'une prothèse vasculaire consiste à réaliser sa mise en communication avec un vaisseau sanguin, par exemple une veine ou une artère. En particulier, elle consiste à réaliser un abouchement de la prothèse et du vaisseau.

Lorsque l'on procède au remplacement d'un tronçon malade d'un vaisseau, on exécute une anastomose termino-terminale, c'est-à-dire que la prothèse est placée dans le prolongement axial de la partie saine du vaisseau, à la place du tronçon malade que l'on élimine.

Dans le cas d'un pontage, on procède à une anastomose termino-latérale, ce qui signifie que l'embouchure de la prothèse est raccordée latéralement au vaisseau, dans lequel on a préalablement pratiqué une lumière latérale à cet effet.

Suivant un premier mode de mise en oeuvre de l'anastomose, la prothèse est cousue au vaisseau à l'aide d'un fil. Dans ce cas, la prothèse vasculaire et le vaisseau sanguin sont cousus bord à bord.

Suivant un second mode de mise en oeuvre de l'anastomose, la prothèse comporte, à son extrémité de raccordement, un rebroussement externe, formant une nappe annulaire dont le bord libre est adapté pour s'appliquer au voisinage immédiat du pourtour de l'ouverture pratiquée dans le vaisseau sanguin. Dans ce cas, l'anastomose de la prothèse vasculaire sur le vaisseau sanguin s'effectue par mise en place d'un ensemble d'attaches, encore dénommées «clip», de manière à solidariser par pincement la nappe annulaire au pourtour de l'ouverture du vaisseau sanguin.

Afin d'assurer une liaison satisfaisante, il est nécessaire de mettre en place des attaches réparties sur le pourtour de l'ouverture environ tous les 4 mm. Chaque attache est mise en place individuellement, de sorte que la durée de l'intervention est longue, ce qui peut être préjudiciable pour la santé du patient, la circulation au travers du vaisseau sanguin devant être interrompue pendant la mise en place de la prothèse.

L'anastomose d'une prothèse vasculaire sur un vaisseau sanguin suivant ce procédé est décrite dans la demande de brevet français n° FR-A-2.751.867 déposée au nom de l'Association René Leriche.

Suivant la méthode décrite dans ce document, il est très difficile de mettre en place successivement chacune des attaches sans devoir augmenter la taille de l'incision. Aussi, cette technique mise en oeuvre dans le cadre de la coelio-chirurgie nécessite beaucoup de temps.

L'invention a pour but de fournir des moyens permettant la réalisation rapide d'une anastomose entre une prothèse tubulaire, par exemple une prothèse vasculaire et un conduit organique tubulaire, par exemple un vaisseau sanguin, notamment dans un champ opératoire limité.

A cet effet, l'invention a pour objet un organe de liaison pour l'anastomose sur un conduit tubulaire muni d'une ouverture latérale ou terminale, d'une prothèse tubulaire comportant, à une extrémité de raccordement, un rebroussement formant une nappe annulaire dont le bord libre est adapté pour s'appliquer au voisinage immédiat du pourtour de l'ouverture du conduit tubulaire, caractérisé en ce qu'il comporte un ensemble d'attaches adaptée chacune pour appliquer la nappe annulaire sur le pourtour de l'ouverture, et en ce qu'il comporte un support annulaire autour duquel sont réparties les attaches.

Suivant des modes particuliers de réalisation, l'organe de liaison comporte l'une ou plusieurs des caractéristiques suivantes :
- ledit support annulaire est venu de matière avec les attaches ;
- ledit support annulaire est amovible pour permettre de désolidariser l'ensemble des attaches ;
- lesdites attaches qui forment pinces comportent deux mâchoires et sont déformables élastiquement, chacune depuis une position de repos dans laquelle l'attache est fermée, les deux mâchoires étant accolées, et une position ouverte permettant sa mise en place dans laquelle les deux mâchoires sont écartées ;
- en position fermée, les attaches s'étendent sensiblement suivant une génératrice du support annulaire ; et
- en position fermée, les attaches sont dirigées sensiblement radialement par rapport au support annulaire.

L'invention a également pour objet un dispositif de mise en place d'attaches pour l'anastomose sur un conduit tubulaire muni d'une ouverture latérale ou terminale d'une prothèse tubulaire comportant, à une extrémité de raccordement un rebroussement formant une nappe annulaire dont le bord libre est adapté pour s'appliquer au voisinage immédiat du pourtour de l'ouverture du conduit tubulaire, caractérisé en ce qu'il comporte un organe de liaison tel que défini ci-dessus et des moyens libérables de retenue de l'ensemble des attaches en position ouverte.

Suivant des modes particuliers de réalisation, le dispositif comporte l'une ou plusieurs des caractéristiques suivantes :
- lesdits moyens libérables de retenue comportent, pour chaque attache, des moyens d'immobilisation d'une première mâchoire par rapport audit support annulaire et des moyens d'immobilisation temporaire de la seconde mâchoire par rapport à la première mâchoire ;
- lesdits moyens d'immobilisation temporaire comportent une bague extérieure rigide de retenue des extrémités des secondes mâchoires de chaque attache ;
- lesdits moyens d'immobilisation temporaire comportent des liens frangibles assurant la liaison entre les secondes mâchoires et ledit support annulaire ;
- lesdits moyens d'immobilisation temporaire comportent un manchon intérieur de retenue des extrémités des secondes mâchoires de chaque attache, lequel manchon est coulissant par rapport audit support annulaire ;
- il comporte un organe de support tubulaire portant à une extrémité ledit support annulaire, lequel support tubulaire délimite un conduit de réception de la prothèse tubulaire, et lesdits moyens libérables de retenue comportent des moyens de libération à distance desdits moyens d'immobilisation temporaire de la seconde mâchoire, lesquels moyens de libération sont montés déplaçables par rapport à l'organe de support tubulaire ;
- les longueurs de l'organe de support tubulaire et des moyens de libération sont suffisantes pour assurer leur actionnement manuel depuis l'extérieur du corps, l'organe de support et lesdits moyens de libération traversant une incision pratiquée sur le corps ;
- lesdits moyens de libération comportent un manche portant à une extrémité ladite bague externe rigide, lequel manche est coulissant par rapport audit organe de support tubulaire pour écarter ladite bague rigide des secondes mâchoires de chaque attache ;
- lesdits moyens de libération comportent au moins un organe coupant porté à l'extrémité d'un manche qui est déplaçable par rapport audit organe de support tubulaire, le ou chaque organe coupant étant adapté pour coopérer avec lesdits liens frangibles ; et
- lesdits moyens de libération comportent un manche prolongeant ledit manchon intérieur, lequel manche est coulissant par rapport audit organe de support tubulaire pour écarter le manchon intérieur des secondes mâchoires de chaque attache.

L'invention a également pour objet un implant comportant une prothèse tubulaire, notamment une prothèse vasculaire, comportant, à une extrémité de raccordement, un rebroussement formant une nappe annulaire dont le bord libre est adapté pour s'appliquer au voisinage immédiat du pourtour de l'ouverture d'un conduit tubulaire sur lequel la prothèse tubulaire doit être anastomosée et un dispositif de mise en place d'attaches tels que défini ci-dessus.

L'invention sera mieux comprise à la lecture de la description qui va suivre, donnée uniquement à titre d'exemple et faite en se référant aux dessins sur lesquels :
- la figure 1 est une vue en coupe d'un dispositif de mise en place d'attaches selon l'invention pour une anastomose termino-terminale, le dispositif étant représenté avant libération des attaches ;
- la figure 2 est une vue en perspective d'un organe de liaison selon l'invention, mis en oeuvre dans le dispositif de la figure 1 ;
- la figure 3 est une vue en élévation d'un tronçon d'un flan découpé dans un feuillard pour la réalisation de l'organe de liaison de la figure 2 ,
- la figure 4 est une vue analogue à celle de la figure1 représentée après libération des attaches ;
- la figure 5 est une vue en perspective éclatée d'une variante de réalisation d'un organe de liaison, en cours de fabrication ;
- la figure 6 est une vue en perspective de l'organe de liaison de la figure 5 au stade suivant de fabrication ;
- la figure 7 est une vue partielle en perspective d'une attache achevée de la variante de réalisation de l'organe de liaison des figures 5 et 6 ;
- la figure 8 est une vue en coupe d'une variante de réalisation du dispositif de mise en place d'attaches de la figure 1 ;
- la figure 9 est une vue partielle en perspective du dispositif de mise en place d'attaches de la figure 8 ;
- la figure 10 est une vue en coupe montrant le dispositif de mise en place d'attaches des figures 8 et 9, après libération des attaches ;
- la figure 11 est une vue en coupe d'un dispositif de mise en place d'attaches selon l'invention pour une anastomose termino-latérale, représenté avant libération des attaches ;
- la figure 12 est une vue en perspective d'une attache utilisée dans le dispositif de la figure 11 ;
- la figure 13 est une vue analogue à celle de la figure 11, le dispositif étant représenté après libération des attaches ;
- la figure 14 est une vue en coupe d'une variante de réalisation d'un dispositif de mise en place d'attaches pour une anastomose termino-latérale ; et
- la figure 15 est une vue en coupe longitudinale d'une autre variante du dispositif de mise en place d'attaches de la figure 1, comportant des moyens de libération à distance des attaches.

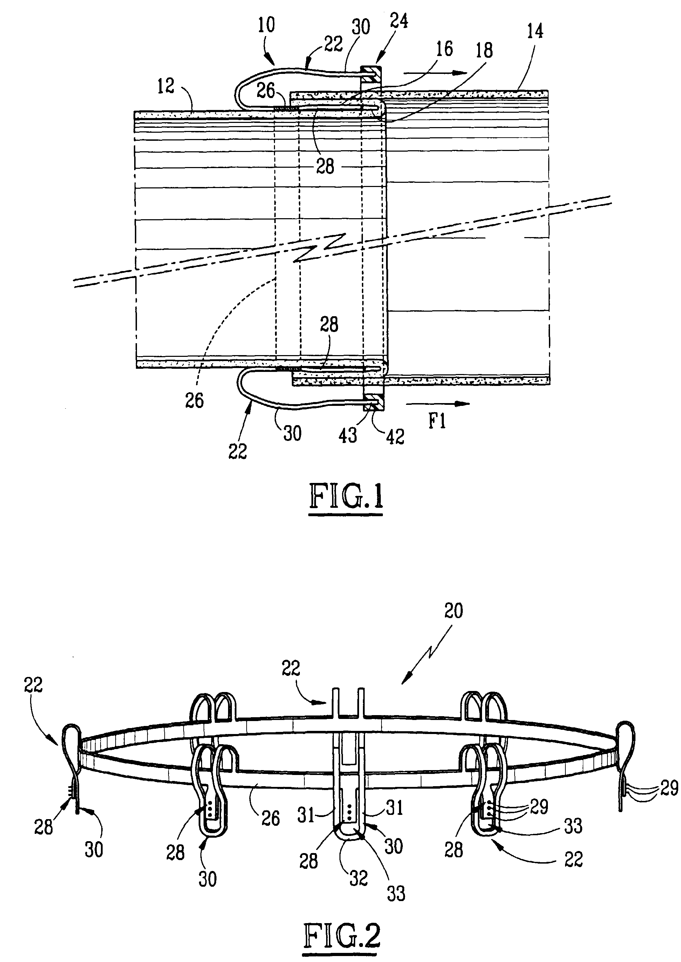

Sur la figure 1 est représenté un dispositif 10 de mise en place d'attaches pour l'anastomose termino-terminale d'une prothèse vasculaire 12 sur un conduit tubulaire 14, par exemple une artère.

La prothèse 12 comporte, à son extrémité de raccordement avec le conduit tubulaire 14, une nappe annulaire externe 16. Celle-ci est formée par le tronçon d'extrémité de la prothèse qui est replié extérieurement et constitue un rebroussement. Ce rebroussement 16 définit une nappe cylindrique entourant extérieurement l'extrémité de la prothèse 12. La nappe délimite ainsi avec la partie courante de la prothèse 12 un canal annulaire 18.

Le dispositif 10 de mise en place d'attaches comporte un organe de liaison 20 portant les attaches, notées 22, et des moyens 24 libérables de retenue en position ouverte de l'ensemble des attaches.

L'organe de liaison seul est représenté à plus grande échelle sur la figure 2.

Dans cet exemple, il comporte huit attaches 22 réparties régulièrement sur le pourtour d'un support annulaire fermé 26. Le support annulaire 26 est constitué par un cerclage métallique avec lequel les attaches 22 sont venues de matière.

Chaque attache 22 comporte deux mâchoires et forme pince. Ces attaches sont chacune déformable élastiquement, entre une position de repos (position de la figure 2), dans laquelle elle est formée, les deux mâchoires étant accolées, et une position ouverte permettant sa mise en place, dans laquelle les deux mâchoires sont écartées.

Chaque attache comporte une première mâchoire 28 destinée à être reçue dans le canal 18 défini entre la partie courante de la prothèse 12 et le rebroussement 16. Cette première mâchoire, dite intérieure, est constituée par une languette plane s'étendant sensiblement parallèlement à une génératrice du support annulaire 26, avec un léger décalage vers l'extérieur. Elle comporte avantageusement trois picots de retenue 29 disposés sur sa surface destinée à s'appliquer sur le remboursement 16.

La seconde mâchoire 30, destinée à s'appliquer élastiquement sur la surface externe du conduit tubulaire 14, présente une forme générale d'arceau en U. Elle est formée par deux branches parallèles 31 venues de matière avec le support annulaire 26 sur le bord opposé à celui portant la mâchoire interne 28. Les deux branches parallèles 31 sont reliées l'une à l'autre par un pontet 32 et délimitent ainsi une lumière 33 de réception de la mâchoire interne 28.

Les deux branches principales 31 sont repliées extérieurement en épingle à cheveux vers et au-delà de la mâchoire interne 28. La distance séparant les deux branches principales parallèles 31 est supérieure à la largeur de la mâchoire interne 28, de sorte que cette dernière est reçue entre les deux branches 31 lorsque l'attache est au repos. L'extrémité de la seconde mâchoire 30 est aplatie et s'étend dans un plan parallèle à celui de la mâchoire interne 28.

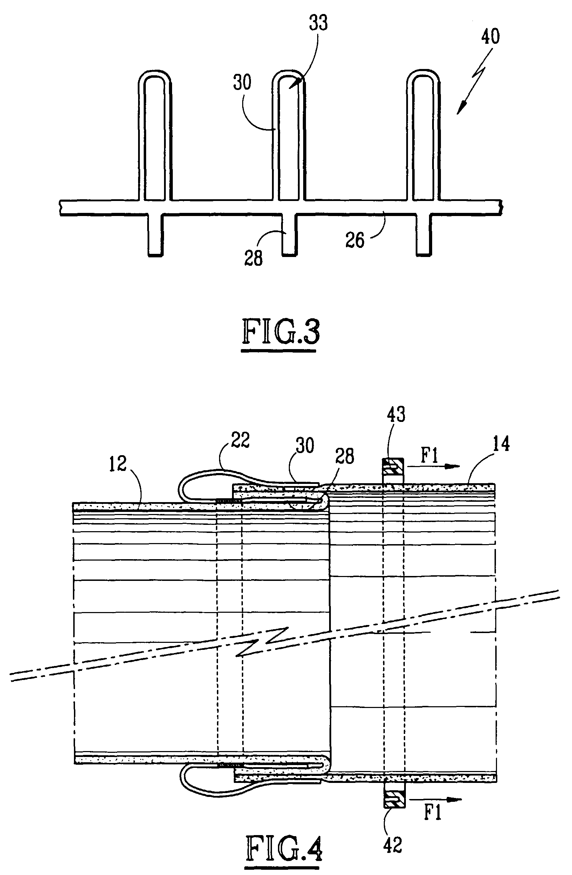

Sur la figure 3, est représenté un tronçon d'un flan 40 destiné à former l'organe de liaison de la figure 2. Ce flan est découpé, par exemple par emboutissage, dans un feuillard métallique plat.

On reconnaît, sur la figure 3, l'ébauche des premières mâchoires 28 et des secondes mâchoires 30 percées des lumières 33 de réception des premières mâchoires.

Afin d'assurer la mise en forme de l'organe de liaison, les secondes mâchoires 30 sont rabattues par déformation plastique vers les premières mâchoires 28 et au-delà de celles-ci. Le support 26 est ensuite configuré en cercle et ses deux extrémités sont soudées bord à bord afin de former un cerclage annulaire. L'ensemble de l'organe de liaison est enfin traité thermiquement afin de conférer leur élasticité aux attaches.

Comme représenté sur la figure 1, les moyens 24 libérables de retenue des attaches en position ouverte comportent des moyens d'immobilisation temporaire de la seconde mâchoire 30 de chaque attache par rapport à la première mâchoire 28, la première mâchoire 28 étant elle-même immobilisée par le support annulaire 26.

Dans le mode de réalisation représenté, les moyens d'immobilisation temporaires comportent une couronne 42 de diamètre intérieur supérieur au diamètre externe du conduit tubulaire 14. La couronne 42 est munie de trous borgnes 43 d'axes parallèles à l'axe de la couronne, destinés à recevoir les extrémités planes des secondes mâchoires 30.

La somme des forces appliquées par les secondes mâchoires élastiques sur la couronne s'annulent, puisque ces forces sont concourantes et de même intensité. La couronne 42 assure ainsi un maintien des attaches 22 en position ouverte et est maintenue sensiblement suivant l'axe du support annulaire 26.

Afin d'assurer l'anastomose de la prothèse 12 à l'extrémité du tronçon tubulaire 14, on insert préalablement les mâchoires internes 28 des attaches dans le canal 18, comme représenté sur la figure 2. L'organe de liaison étant associé à une couronne 24 de retenue des attaches en position ouverte, la mise en place de l'organe de liaison entre la partie courante de la prothèse 12 et le rebroussement 16 est aisée.

La prothèse 12 ainsi équipée du dispositif de mise en place d'attaches est ensuite engagée à l'extrémité du conduit tubulaire 14. En particulier, le rebroussement 16 est introduit à l'intérieur du conduit tubulaire 14, alors que la couronne 42 et les secondes mâchoires 30 sont disposées autour de l'extrémité du conduit tubulaire 14.

Afin d'assurer la liaison de la prothèse 12 à l'extrémité du conduit 14, la couronne 42 est seule déplacée le long du conduit 14 suivant le sens de la flèche F1.

Comme représenté sur la figure 3, le déplacement de la couronne 42 produit la libération des extrémités des mâchoires extérieures 30. Celles-ci se rabattent alors sur la face externe du conduite tubulaire 14, assurant ainsi le pincement de celui-ci et du rebroussement 16 entre les deux mâchoires des attaches.

La couronne 42 est ensuite extraite, soit par coulissement jusqu'à l'extrémité libre du tronçon tubulaire 14 ou par sectionnement à l'aide d'une pince adaptée.

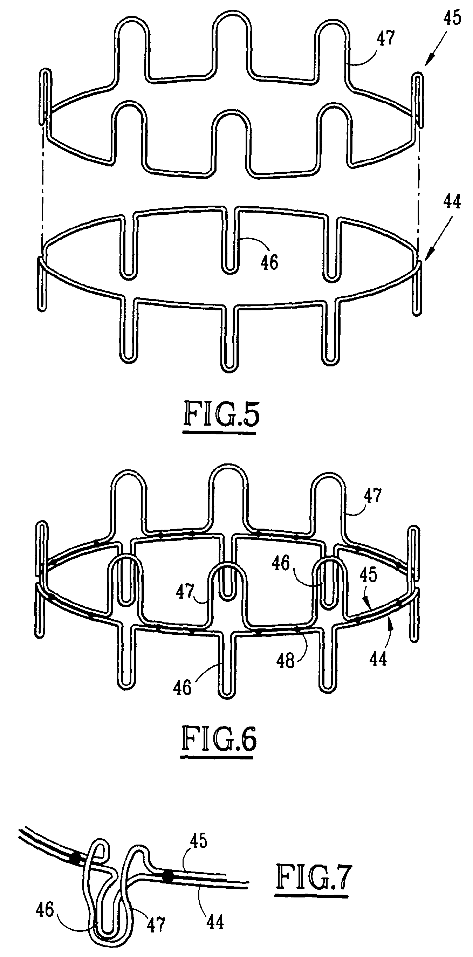

Sur les figures 5 à 7 est représentée une variante de réalisation de l'organe de liaison selon l'invention. Dans ce mode de réalisation, celui-ci est constitué par deux fils 44, 45 conformés en anneaux comportant des lobes 46, 47 définissant chacun une mâchoire.

Comme représenté sur la figure 6, les deux fils 44,45 sont solidarisés l'un sur l'autre par des soudures 48 avec leurs lobes associés en regard. Les lobes 46, 47 sont ensuite déformés, comme dans le mode de réalisation de la figure 2, afin de définir les mâchoires élastiques des attaches. Ces dernières sont visibles sur la figure 7.

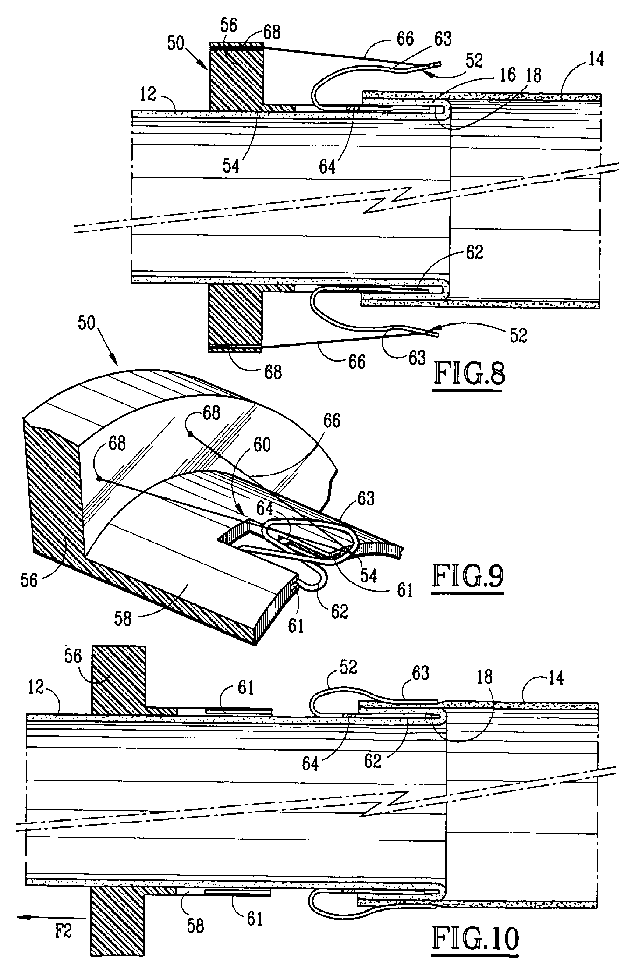

Sur la figure 8 est représentée une variante du dispositif de mise en place d'attaches. Celui-ci comporte un manchon 50 formant support annulaire sur la périphérie duquel sont réparties des et notamment huit attaches filiformes indépendantes 52. Le manchon 50 comporte un passage cylindrique interne 54 dont le diamètre est légèrement supérieur au diamètre externe de la prothèse 12. Il comporte extérieurement, à l'arrière, une collerette périphérique externe 56. A l'avant, le manchon 50 définit un collet 58 de faible épaisseur destiné à être reçu dans le canal 18 délimité entre la partie courante de la prothèse 12 et le rebroussement 16.

Comme représenté sur la figure 9, les attaches 52 sont reçues dans des encoches axiales 60 régulièrement réparties sur le collet 58 et débouchant à l'extrémité avant du manchon. Ces encoches 58 ont une forme sensiblement rectangulaire et présentent sur leur paroi latérale, dans l'épaisseur du collet 58, des rainures axiales 61 de maintien et de guidage des attaches 52.

Ces attaches ont des formes analogues à celles représentées sur les figures 2 et 7, c'est-à-dire qu'elles comportent deux mâchoires 62, 63 sollicités élastiquement l'une vers l'autre. Celles-ci ne sont toutefois pas liées les unes aux autres par un support annulaire venu de matière. En revanche, des ailes latérales 64 destinées à être reçues dans les fentes 52 sont prévues sur les attaches de part et d'autre de la zone de liaison entre les deux mâchoires 62, 63. Ces ailes 64 sont formées par des pattes planes de largeur suffisante pour interdire la rotation des attaches autour de celle-ci.

Ainsi, comme représenté sur la figure 9, les ailes 64 reçues dans les rainures 61 permettent une retenue et un guidage des attaches 52 avec leur mâchoire interne s'étendant suivant le prolongement du manchon.

Afin d'assurer le maintien temporaire de chaque attache 52 en position ouverte, la mâchoire externe 63 de celle-ci est retenue écartée de la mâchoire interne 62 par un lien frangible 66 passant au travers de l'attache. Ce lien est repris, de part et d'autre, au travers de passages 68 traversant axialement de part en part la collerette 56.

On conçoit ainsi qu'un unique lien 66 permet par des passages successifs au travers de la collerette 56 la retenue en position écartée de toutes les mâchoires externes des attaches portées par le manchon.

Afin de faciliter le passage et la retenue du lien à l'extrémité de la machine externe, celle-ci comporte un profil en creux adapté non représenté.

Comme visible sur la figure 8, afin d'assurer la liaison de la prothèse 12 au conduit tubulaire 14, le manchon 50, portant les attaches 52, est disposé autour de la prothèse, le collet 58 et les mâchoires internes 62 des attaches étant disposés dans le canal 18. Les liens 66 retenant écartées les mâchoires externes 63, celles-ci s'étendent, avant sectionnement du lien 66, immédiatement au-dessus de la surface externe du conduit tubulaire 14.

Après sectionnement du lien, les mâchoires externes 63 se rabattent sous l'action de l'élasticité propre de chaque attache afin d'assurer le pincement de l'extrémité du conduit 14 et du rebroussement 16. Enfin, après libération des attaches, le manchon 50 est extrait par déplacement le long de la prothèse 12 suivant le sens de la flèche F2, comme représenté sur la figure 10. Si nécessaire, le manchon 50 est sectionné afin d'être retiré.

Dans ce mode de réalisation, les attaches, après réalisation de l'anastomose, sont indépendantes les unes des autres. Ainsi, la zone de liaison entre le conduit tubulaire et la prothèse est libre de s'étendre radialement, notamment lors des pulsations cardiaques augmentant temporairement le débit du flux sanguin.

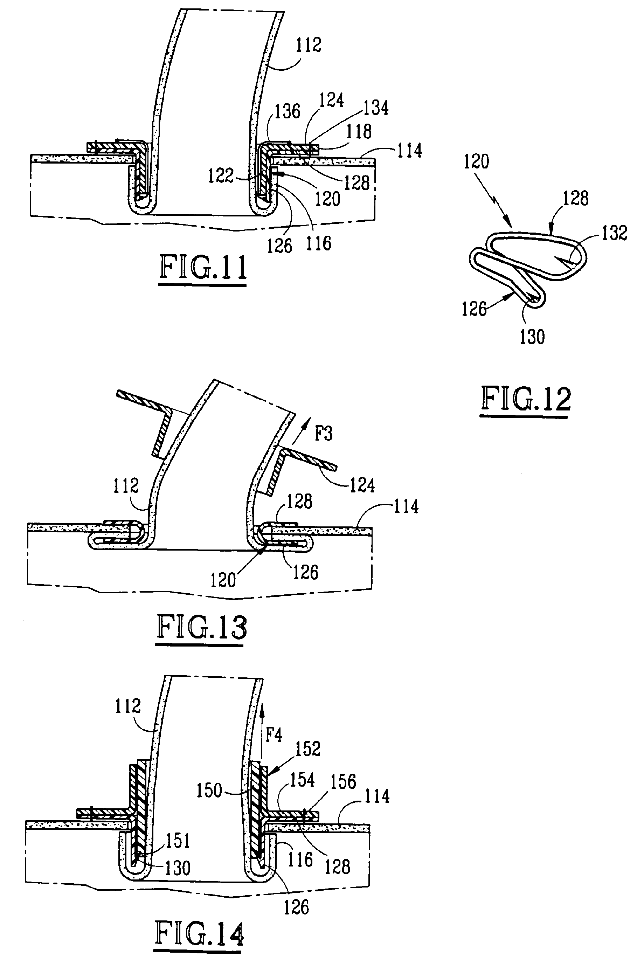

Sur la figure 11 est représenté un dispositif de mise en place d'attaches pour la réalisation d'une anastomose termino-latérale.

Dans le cas d'une telle anastomose, la prothèse, notée 112, est destinée à être solidarisée à un conduit tubulaire 114 muni d'une ouverture latérale sensiblement circulaire. La prothèse 112 comporte, à son extrémité de raccordement, un rebroussement 116 constituant une nappe dont le bord est destiné à venir s'appliquer intérieurement suivant le pourtour de l'ouverture ménagée dans le conduit 114.

Pour la réalisation de telles anastomoses, le dispositif de mise en place d'attaches comporte un support annulaire 118 à la périphérie duquel sont disposées des attaches élastiques 120.

Le support 118 est constitué par un anneau en plastique rigide ayant une section transversale en équerre. Plus précisément, il comporte un tronçon tubulaire 122 destiné à s'engager, lors de la mise en place, entre la partie courant de la prothèse 112 et le rebroussement 116. Il comporte en outre dans le prolongement du tronçon tubulaire 122 une collerette externe 124.

Les attaches 120 sont constituées par des pinces élastiques telles que celle représentée par exemple sur la figure 12. Elles sont formées par un fil refermé en anneau et conformé, comme expliqué précédemment, pour définir une mâchoire interne 126 et une mâchoire externe 128. L'élasticité de l'attache est suffisante pour permettre un décalage angulaire des deux mâchoires l'une par rapport à l'autre de l'ordre de 90°, tout en permettant que, après libération, les deux mâchoires reviennent en position de repos sensiblement dans le même plan.

De plus, les mâchoires 126, 128 comportent chacune à l'intérieur du rebroussement constituant leur extrémité, un plot, noté respectivement 130, 132, pour le passage de liens de maintien de l'attache en position ouverte.

Comme visible sur la figure 11, les attaches sont maintenues initialement en position ouverte dans l'angle aigu délimité entre le tronçon tubulaire 122 et la collerette 124.

A cet effet, un lien 134 traversant axialement la collerette 124 plaque la mâchoire externe 128 de l'attache contre la collerette. Le lien 134 passe à cet effet autour du plot 132.

De même, un lien 136, dont une extrémité est engagée au niveau du plot 130, s'étend le long de la paroi cylindrique intérieure du tronçon 122 et est solidarisé sur la face dégagée de la collerette 124. Ainsi, les attaches sont maintenues en position ouverte contre le support annulaire 118.

Pour la mise en place de la prothèse, la mâchoire interne 126 des attaches, plaquée contre le tronçon tubulaire 122, est engagée dans le canal délimité entre la partie courant de la prothèse 112 et le rebroussement 116. L'ensemble ainsi formé constitue un implant vasculaire qui est avantageusement commercialisé pré-assemblé. La prothèse, ainsi équipée du dispositif de mise en place des attaches, est ensuite introduite au travers de l'ouverture latérale ménagée dans la conduite 114.

Alors que les mâchoires externes des attaches sont appliquées sur la surface externe de la conduite tubulaire 114, les liens 136 sont sectionnés, libérant ainsi les mâchoires internes 126 qui, sous l'effet de l'élasticité des attaches, viennent plaquer le rebroussement 116 contre la surface interne de la conduite 114.

Les liens 134 sont ensuite sectionnés, afin de permettre à la bague 124 d'être retirée par coulissement suivant le sens de la flèche F3, comme représenté sur la figure 13.

Sur la figure 14, est représenté encore un autre mode de réalisation du dispositif de mise en place d'attaches pour la réalisation d'une anastomose termino-latérale. Dans ce mode de réalisation, les attaches sont analogues à celles du mode de réalisation des figures 11 à 13.

Le support des attaches est constitué de deux parties coulissantes. Une première partie, destinée à maintenir la mâchoire interne 126, est constituée d'un manchon de section ovalaire et de faible épaisseur, noté 150. Ce manchon comporte, à l'une de ses extrémités, dans son épaisseur, des évidements axiaux 151 de réception des plots 130.

La seconde partie, notée 152, est formée par une bague de section en équerre. Le diamètre intérieur de la bague est égal au diamètre externe du manchon 150, de sorte que ce dernier peut coulisser à l'intérieur de la bague 152.

Suivant sa branche s'étendant radialement, notée 154, la bague 152 comporte des liens frangibles 156 de retenue de la mâchoire externe 128 des attaches.

Le manchon 150 et la mâchoire interne 126 sont disposés dans le canal délimité par la partie courante de la prothèse et le rebroussement 116. L'extrémité de la prothèse est introduite dans l'ouverture latérale de la conduite 114, alors que la mâchoire externe est appliquée sur la surface latérale externe de la conduite.

Le manchon 150 est ensuite déplacé à coulissement suivant le sens de la flèche F4 à l'intérieur de la bague 152. Le déplacement du manchon 150 conduit à la libération de la mâchoire interne 126 qui vient plaquer le rebroussement 116 sur la surface intérieure du conduit 114. Les liens frangibles 156 sont ensuite coupés de sorte que les attaches sont libérées et la bague 152 peut être retirée.

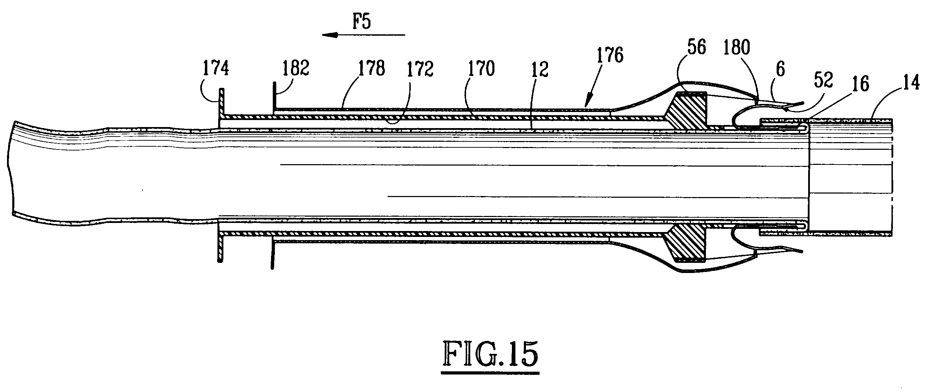

Sur la figure 15 est représentée une variante de réalisation du dispositif de mise en place d'attaches de la figure 8. Les éléments analogues y sont désignés par les mêmes références.

Dans cette variante, le manchon 50 est prolongé autour de la prothèse 12 par un prolongement tubulaire 170 formant un organe de support. Ce dernier est avantageusement constitué d'un ressort à spires jointives. Il définit un conduit 172 au travers duquel passe la partie courante de la prothèse 12. A son extrémité libre opposée au manchon 50, le prolongement 170 présente une collerette 174 permettant sa commande manuelle. La longueur du prolongement 172 est suffisante pour permettre la mise en place de l'organe de liaison à l'intérieur du corps, la collerette 174 étant maintenue à l'extérieur du corps.

Dans ce mode de réalisation, les moyens libérables de retenue de l'ensemble des attaches en position ouverte comportent des moyens 176 de libération à distance des moyens d'immobilisation temporaires 66 formés ici par des liens frangibles. Ces moyens de libération 176 comportent un manche tubulaire 178 monté déplaçable à coulissement axial à l'extérieur du prolongement tubulaire 172. Le manche 178 porte, à son extrémité voisine des attaches, des organes coupants 180 adaptés pour coopérer avec les liens frangibles 66. Ces organes coupants sont par exemple des lames effilées appliquées contre les liens frangibles 66.

A son autre extrémité, le manche 178 comporte une collerette 182 pour son actionnement manuel. La longueur du manche 178 est suffisante pour permettre un actionnement des moyens de libération 176 depuis l'extérieur du corps du patient, alors que l'organe de liaison est positionné dans le corps du patient.

Avec un tel dispositif, il est possible de libérer les attaches 52 par une simple traction sur le manche 178, suivant le sens de la flèche F5, le support tubulaire 170 étant maintenu fixe. Cette traction provoque le coulissement des moyens de libération 176 par rapport au prolongement 172. Ce coulissement entraîne la rupture des liens frangibles 166 sous l'action des organes coupants 180.

Après libération des attaches, le manchon 50, solidaire du support tubulaire 170, et les moyens de libération 176 sont extraits par l'incision pratiquée sur le patient, seule la prothèse et l'organe de liaison restant en place.

Le dispositif représenté sur les figures 1 et 14 peut également être muni de moyens de libération à distance des moyens d'immobilisation temporaire des secondes mâchoires des organes de liaison. Pour ce faire, la bague rigide 42 ou le manchon 150 sont respectivement fixés à l'extrémité d'un manche rigide. Ce bras rigide est monté coulissant autour d'un prolongement tubulaire prévu autour de la prothèse, lequel prolongement supporte l'organe de liaison et le maintient en position.

La description faite ici présente des moyens pour l'anastomose d'une prothèse vasculaire sur une artère ou une veine. Toutefois, ces moyens peuvent être mis en oeuvre pour tout type de prothèse tubulaire afin d'assurer leur liaison sur un conduit tubulaire organique tel que le tube digestif ou l'uretère.

## Revendications

1. Organe de liaison (20) pour l'anastomose sur un conduit tubulaire (14, 114) muni d'une ouverture latérale ou terminale, d'une prothèse tubulaire (12, 112) comportant, à une extrémité de raccordement, un rebroussement (16, 116) formant une nappe annulaire dont le bord libre est adapté pour s'appliquer au voisinage immédiat du pourtour de l'ouverture du conduit tubulaire, caractérisé en ce qu'il comporte un ensemble d'attaches (22 ; 52 ; 120) adaptée chacune pour appliquer la nappe annulaire (16, 116) sur le pourtour de l'ouverture, et un support annulaire (26 ; 50 ; 118 ; 152) autour duquel sont réparties les attaches (22 ; 52 ; 120), et en ce que lesdites attaches (22 ; 52 ; 120) qui forment pinces comportent deux mâchoires (28, 30 ; 46, 47 ; 62, 63 ; 126, 128) et sont déformables élastiquement, chacune depuis une position de repos dans laquelle l'attache est fermée, les deux mâchoires étant accolées, et une position ouverte permettant sa mise en place dans laquelle les deux mâchoires sont écartées.

2. Organe de liaison selon la revendication 1, caractérisé en ce que ledit support annulaire (26) est venu de matière avec les attaches (22).

3. Organe de liaison selon la revendication 1, caractérisé en ce que ledit support annulaire (50 ; 118 ; 152) est amovible pour permettre de désolidariser l'ensemble des attaches (52 ; 120).

4. Organe de liaison selon l'une quelconque des revendications précédentes, caractérisé en ce que, en position fermée, les attaches (22 ; 52) s'étendent sensiblement suivant une génératrice du support annulaire (26 ; 50).

5. Organe de liaison selon l'une quelconque des revendications précédentes, caractérisé en ce que, en position fermée, les attaches (120) sont dirigées sensiblement radialement par rapport au support annulaire (118 ; 152).

6. Dispositif de mise en place d'attaches pour l'anastomose sur un conduit tubulaire muni (14 ; 114) d'une ouverture latérale ou terminale d'une prothèse tubulaire (12 ; 112) comportant, à une extrémité de raccordement, un rebroussement (16 ; 116) formant une nappe annulaire dont le bord libre est adapté pour s'appliquer au voisinage immédiat du pourtour de l'ouverture du conduit tubulaire (14 ; 114), caractérisé en ce qu'il comporte un organe de liaison (20) selon l'une quelconque des revendications 4 à 6 et des moyens (24 ; 66 ; 136 ; 150) libérables de retenue de l'ensemble des attaches en position ouverte.

7. Dispositif selon la revendication 6, caractérisé en ce que lesdits moyens libérables de retenue comportent, pour chaque attache, des moyens (26 ; 64 ; 124 ; 134 ; 154 , 156) d'immobilisation d'une première mâchoire (28 ; 62 ; 128) par rapport audit support annulaire (26 ; 50 ; 118 ; 152) et des moyens (42 ; 50, 66 ; 122, 136 ; 150) d'immobilisation temporaire de la seconde mâchoire (30 ; 63 ; 126) par rapport à la première mâchoire (28 ; 62 ; 128).

8. Dispositif selon la revendication 7, caractérisé en ce que lesdits moyens (24) d'immobilisation temporaire comportent une bague extérieure rigide (42) de retenue des extrémités des secondes mâchoires (30) de chaque attache (22).

9. Dispositif selon la revendication 7, caractérisé en ce que lesdits moyens d'immobilisation temporaire comportent des liens frangibles (66 ; 136) assurant la liaison entre les secondes mâchoires (63 ; 126) et ledit support annulaire (50 ; 118).

10. Dispositif selon la revendication 7, caractérisé en ce que lesdits moyens d'immobilisation temporaire comportent un manchon intérieur (150) de retenue des extrémités des secondes mâchoires (126) de chaque attache, lequel manchon est coulissant par rapport audit support annulaire (152).

11. Dispositif selon l'une quelconque des revendications 7 à 10, caractérisé en ce qu'il comporte un organe de support tubulaire (170) portant à une extrémité ledit support annulaire (26 ; 50 ; 118 ; 152), lequel support tubulaire (170) délimite un conduit (172) de réception de la prothèse tubulaire (12 ; 112), et en ce que lesdits moyens libérables de retenue comportent des moyens (176) de libération à distance desdits moyens (42 ; 50, 66 ; 122, 136 ; 150) d'immobilisation temporaire de la seconde mâchoire (28 ; 62 ; 128), lesquels moyens de libération (176) sont montés déplaçables par rapport à l'organe de support tubulaire (170).

12. Dispositif selon la revendication 11, caractérisé en ce que les longueurs de l'organe de support tubulaire (170) et des moyens de libération (176) sont suffisantes pour assurer leur actionnement manuel depuis l'extérieur du corps, l'organe de support (170) et lesdits moyens de libération (176) traversant une incision pratiquée sur le corps.

13. Dispositif selon la revendication 11 ou 12 prise avec la revendication 8, caractérisé en ce que lesdits moyens de libération comportent un manche portant à une extrémité ladite bague externe rigide (42), lequel manche est coulissant par rapport audit organe de support tubulaire pour écarter ladite bague rigide (42) des secondes mâchoires (30) de chaque attache.

14. Dispositif selon la revendication 12 ou 13 prise avec la revendication 9, caractérisé en ce que lesdits moyens de libération (176) comportent au moins un organe coupant (180) porté à l'extrémité d'un manche (178) qui est déplaçable par rapport audit organe de support tubulaire (178), le ou chaque organe coupant (180) étant adapté pour coopérer avec lesdits liens frangibles (66 ; 136).

15. Dispositif selon la revendication 11 ou 12 prise avec la revendication 10, caractérisé en ce que lesdits moyens de libération comportent un manche prolongeant ledit manchon intérieur (150), lequel manche est coulissant par rapport audit organe de support tubulaire pour écarter le manchon intérieur (150) des secondes mâchoires (126) de chaque attache.

16. Implant comportant une prothèse tubulaire (12 ; 112), notamment une prothèse vasculaire, comportant, à une extrémité de raccordement, un rebroussement (16 ; 116) formant une nappe annulaire dont le bord libre est adapté pour s'appliquer au voisinage immédiat du pourtour de l'ouverture d'un conduit tubulaire (14 ; 114) sur lequel la prothèse tubulaire (12 ; 112) doit être anastomosée et un dispositif de mise en place d'attaches selon l'une quelconque des revendications 6 à 15.
